# EUROPEAN PATENT APPLICATION

(11) **EP 4 136 965 A1**
(43) Date of publication of application: **22.02.2023**
(21) Application number: 22191685.1
(22) Date of filing: 27.02.2017
(51) Int. Cl.: A01K 11/00, A61B 10/02, A61B 10/00

(54) **TISSUE SAMPLING CARTRIDGE, METHOD OF TAKING AND STORING A SAMPLE, AND KIT**

(30) Priority: 08.03.2016 US 201662305498 P; 31.03.2016 NZ 16718587; 31.03.2016 NZ 16718588; 31.03.2016 US 201662316499 P; 29.04.2016 NZ 16719578; 02.11.2016 NZ 16725834; 02.11.2016 NZ 16725835; 02.11.2016 NZ 16725836; 02.11.2016 NZ 16725837
(62) Divisional of application: 17762608.2
(71) Applicant: Snpshot Trustee Limited, Auckland 1010 (NZ)
(72) Inventor: BLADEN, Rory, Chicago (US); BLADEN, Roy Victor, Auckland (NZ); GARDNER, Michael Stuart, Auckland (NZ)
(74) Representative: Algemeen Octrooi- en Merkenbureau B.V.

(57) **Abstract**

The present invention relates to a sampling cartridge comprising a sampling cartridge body. The sampling cartridge body includes a storage container holding region, at where a storage container is or is able to be located. It also includes a sample collector holding region at where a sample collector is or is able to be located.

In use the body holds the sample collector and storage container in alignment with each other in a pre-sampling condition spaced apart with a sufficient gap to allow part of an item to be sampled to locate at said gap.

## Description

### FIELD OF THE INVENTION

The present invention relates to tissue sampling, and in particular though not solely to a cartridge carrying components to facilitate the removal of a sample from an animal.

### BACKGROUND TO THE INVENTION

Tissue sampling for the purposes of recording an animal's DNA and for subsequent tracking purposes is becoming more prevalent. For example, farmers may remove a tissue sample from the ear of an animal on the farm. The removed sample may then be in some way associated with the animal by way of a unique identifier, and may then be shipped to a laboratory for analysis. However, difficulties are still arising in respect of sample security and the prevention of tampering of the sample on the farm or during transit to the laboratory, as well as issues around cross-contamination of the sample between animals.

It is therefore an object of the present invention to provide a sampling cartridge and/or sampler with which the cartridge is removably associated that overcomes the abovementioned disadvantages, and/or at least provides the public with a useful choice.

Where used in this specification tissue means any part of a living thing, particularly any part made up of similar cells, or any part or parts that perform a similar function. Tissue preferably refers to any form of biological sample, from plants and animals particularly, including pigs, goats, cattle, sheep, poultry, and fish. Biological samples may include for example, animal tissue such as flesh, blood, hair, fur, saliva, sweat, urine, etc., or plant tissue such as leaves, bark, roots or wood, or any other part of a plant or animal but particularly those that are made up of similar cells, or which perform a similar function.

The cartridge of the present invention may be used in tissue sampling at least for either or both of production animals and companion animals. It is anticipated that production animals may include but not be limited to bovine, pigs, deer and sheep. Further it is anticipated that companion animals may include but not be limited to horses, cats and dogs.

Accordingly in a first aspect the present invention may broadly be said to be a sampling cartridge comprising a sampling cartridge body that includes a storage container holding region at where a storage container is or is able to be located and a sample collector holding region at where a sample collector is or is able to be located, wherein the body in use holds the sample collector and storage container in alignment with each other in a pre-sampling condition spaced apart with a sufficient gap to allow part of an item to be sampled to locate at said gap.

Preferably the cartridge is adapted and configured to be loadable and unloadable to and from a sampler device that includes a driver actuable to cause said in alignment sample collector to move from sample collector holding region across the gap and into the sample storage container.

Preferably the sampling cartridge body is adapted and configured to shield the sampler device at the gap from contact with said item to be sampled when placed at the gap.

Preferably the sampling cartridge body is adapted and configured to shield the sampler device at the gap from contact with said item to be sampled when placed at the gap, and any exudates expressed or expelled from said item during sampling.

Preferably the cartridge comprising a bridge section extending between said storage container holding region and said sample collector holding region, said bridge section spanning said gap.

Preferably said bridge section provides said shield function.

Preferably said bridge section is the only connection of the cartridge between said storage container holding region and said sample collector holding region.

Preferably said bridge section defines a trough to said gap.

Preferably the gap is substantially U shaped, including two facing side walls and a base defined by said bridge section and said storage container holding region and said sample collector holding region.

Preferably the gap is substantially U shaped.

Preferably said storage container holding region and said sample collector holding region are contiguous respective opposed sides of said gap.

Preferably said storage container holding region comprises a cavity of said sampling cartridge body in which at least part of said storage container is located.

Preferably said cavity is a bore.

Preferably a passage leads from said cavity to said gap and via which sample collector can move to engage with said storage container.

Preferably the passage is blocked pre-sampling.

Preferably the passage is blocked by a removable seal.

Preferably the removable seal is a film, preferably able to be ruptured and/or peeled away from the passage to expose the passage to said gap.

Preferably the passage includes a mouth opening presented at said gap at where said sample collector enters the passage, upon the taking of a sample from an item to be sampled placed at the gap, to advance for engagement via on opening of and into said storage container.

Preferably the material about said mouth is harder than said material of the storage container about its said opening.

Preferably at said mouth opening said sampling cartridge body together with said sample collector act as a punch and die set, the mouth opening acting as the die acting in shear with the sample collector acting as said punch to facility the removal of a sample from the item to be sampled.

Preferably said mouth opening is of a shape and configuration to allow said sample collector to enter into said passage in a snug fit manner.

Preferably passage extends between its said opening and said cavity at or adjacent where an opening of said storage container is presented.

Preferably said cavity is closed by a removable closure that together with said cavity encloses said storage container in said sampling cartridge body.

Preferably said removable closure is able to be removed to open said cavity and expose said storage container to allow said storage container to be removed from said sampling cartridge body.

Preferably said removable closure is engaged to said sampling cartridge body in a tamper evident manner.

Preferably said removable closure is threadingly engaged to said sampling cartridge body.

Preferably said sampling cartridge body includes a second cavity in which said sample collector is at least partially located, pre-sampling.

Preferably said sample collector is entirely located in said second cavity.

Preferably said second cavity includes an opening to said gap via which said sample collector can pass from said cavity towards said storage container during sampling.

Preferably said opening of said second cavity is sealed by a removable seal (e.g. a film seal of cap or plug).

Preferably said second cavity is fully sealed pre sampling to *enclose* said sample collector therein.

Preferably the body carries only one sample collector and one sample storage container.

Preferably at least one of the components selected from the following list:
a. Removable closure,
b. Storage container (preferably the storage tube),
c. Sample collector (preferably the plunger)
d. Sampling cartridge body, comprises at least one form of visible and/or machine readable identification.

Preferably the identification is a unique identification.

Preferably the identification is not the same on each of said components but are preferably matched.

Preferably the identification is provided in the form selected from one of more of an electronic identification (EID) or visible identification such as a bar code, number, QR code, alphanumeric string or the like.

Preferably the sample collector comprises a punch presenting at one end a cutter to cut and hold a sample from the item as it passes there through, the punch having a bore at where a sample, once cut is able to locate with said punch.

Preferably said sample collector further comprises a plunger slideably supported at said bore to be able to move between a retracted position and an advanced position relative said punch, the movement from the retracted position to the advanced position causing, in use, the cutter held sample to be displaced from the cutter.

Preferably the sample collector is generally of circular cross section and elongate and straight, the cutter located at one end of the collector.

Preferably the punch is generally of circular cross section and elongate and straight, the cutter located at one end of the collector.

Preferably the plunger protrudes from the end of the punch opposite the cutter.

Preferably the plunger is rod shaped.

Preferably the punch is to seal the opening (or passage extending therefrom) of a sample storage container with which the sample collector is to engage after the sample has been taken.

Preferably the storage container includes a mouth opening leading to a storage region of the storage container, the mouth opening able to receive the sample collector after the sample collector has been driven through the item and is carrying a sample.

Preferably the storage container is adapted and configured to retain the sample collector (preferably the punch) once received at said mouth opening in a manner to snugly hold the sample collector to said storage container (preferably sealing the mouth opening).

Preferably said storage container (preferably at or adjacent said mouth opening) includes a surface or surfaces that allow the sample collector to seal the containment region of the storage container.

Preferably said storage container comprises a container body defining a containment region and a cap removably engaged to the container body, a mouth opening to said container body provided at a passage defined by said cap.

Preferably said passage and said sample collectors are adapted and configured to engage in a snap-fit manner to retain the sample collectors with the cap and seal the containment region.

In a further aspect the present invention may be said to be a method of taking and storing a sample from an item using the sampling cartridge as herein before described comprising:
a. loading the sampling cartridge to a sampler device so that the sample collector and storage container are in alignment with a driver of said sampler device, the driver to act, directly or indirectly on said sample collector,
b. placing a part of an item to be sampled in said gap so that part of the item is also in alignment,
c. causing the driver to move in a driving direction to drive said sample collectors from its holding region, across the gap and towards said storage container so that said sample collector is driven into the storage container so that said punch becomes held by said storage container,
d. causing the driver to move in a direction opposite said driving direction.

Preferably said identification carried by or of the item from which the sample is taken is recorded at the time of taking of the sample.

Preferably the identification is matched to identification of said sampling cartridge.

Preferably said sampling cartridge is removed from the sampling device after the sample has been removed from the item to be taken, the sampling cartridge carrying the sample collector engaged with the storage container at one side of the gap.

Preferably the removed sampling cartridge is then sent to a laboratory for sample analysis.

Preferably the identification and the item and the identification of the sampling cartridge are reviewed after sampling to determine these correspond to the matched identification.

In a further aspect the present invention may be said to be a kit of a sampler and sampling cartridge as herein before described wherein the sampler comprises an actuable driver and a receptacle configured to releaseably receive said cartridge to said sampler said driver presented and actuable to cause said in alignment sample collector to move from sample collector holding region across the gap and into the sample storage container.

Preferably the sampler comprises a U shape receptacle at where the cartridge is receivable.

Preferably the sampling cartridge body is adapted and configured to shield the sampler at the gap from contact with said item to be sampled when placed at the gap, and any exudates expressed or expelled from said item during sampling.

In a further aspect the present invention may be said to be a sampler for use with the cartridge as herein before described.

Preferably the sampling cartridge body is disposable (and is preferably disposed of after the same retaining storage container has been removed from it).

Preferably the sampling cartridge engages with said sampling device in a releasable snap fit or other locked manner.

In yet a further aspect the present invention may broadly be said to be, as a set, a sampling device and a plurality of sampling cartridges as herein before described, each individually and successively loadable with said sampling device for the purposes of each collecting one sample from one of more items to be sampled.

Preferably one (or more) of the invention(s) herein described may be used for production animals and for companion animals.

Preferably one (or more) of the invention(s) herein described may only be used for production animals.

Preferably one (or more) of the invention(s) herein described may only be used for companion animals.

Preferably production animals include but are not limited to bovine, pigs, deer and sheep.

Preferably companion animals include but are not limited to horses, cats and dogs.

In this specification where reference has been made to patent specifications, other external documents, or other sources of information, this is generally for the purpose of providing a context for discussing the features of the invention. Unless specifically stated otherwise, reference to such external documents is not to be construed as an admission that such documents, or such sources of information, in any jurisdiction, are prior art, or form part of the common general knowledge in the art.

The term "comprising" as used in this specification means "consisting at least in part of'. When interpreting each statement in this specification that includes the term "comprising", features other than that or those prefaced by the term may also be present. Related terms such as "comprise" and "comprises" are to be interpreted in the same manner.

This invention may also be said broadly to consist in the parts, elements and features referred to or indicated in the specification of the application, individually or collectively, and any or all combinations of any two or more of said parts, elements or features, and where specific integers are mentioned herein which have known equivalents in the art to which this invention relates, such known equivalents are deemed to be incorporated herein as if individually set forth.

The invention consists in the foregoing and also envisages constructions of which the following gives examples only.

### BRIEF DESCRIPTION OF THE DRAWINGS

A preferred form of the invention will now be described with reference to the accompanying drawings in which:
Figure 1 is a perspective view of a sampler device and sampling cartridge associated therewith,
Figure 2 is an alternative perspective view of a sampler device and sampling cartridge associated therewith,
Figure 3 is a view of the sampling device and sampling cartridge in a pre- or post-engaged condition showing the removable nature of the sampling cartridge,
Figure 4 is a perspective view of Figure 3,

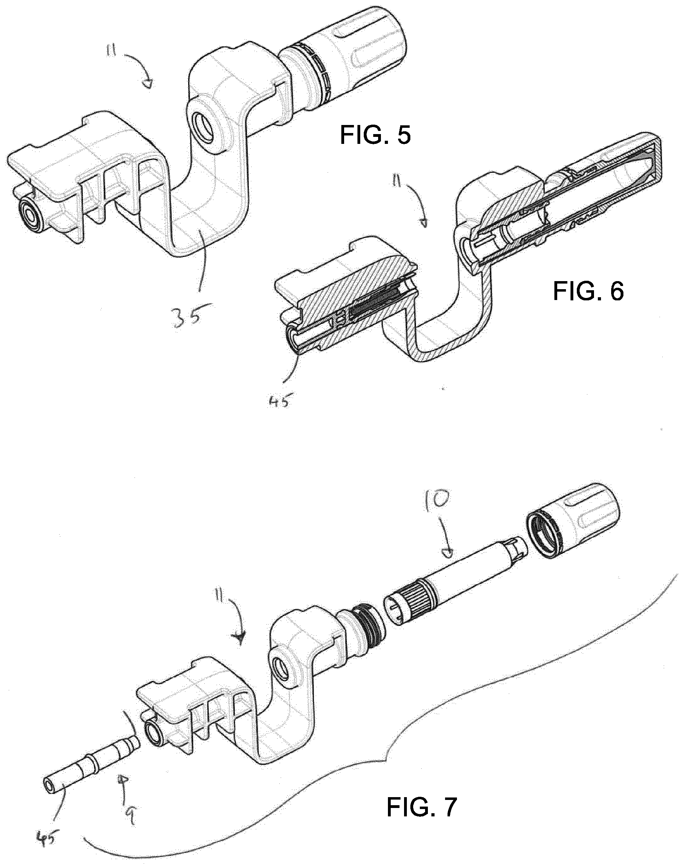

Figure 5 is a perspective view of the sampling cartridge,
Figure 6 is a perspective and partial sectional view of the sampling cartridge,
Figure 7 is an exploded view of the sampling cartridge,
Figure 8 is a sectional view of the sampling cartridge in a preloaded and ready for use condition,
Figure 9 is a sectional view of the sampling cartridge in a condition where the sample collector has advanced from its stored condition towards its sample storage container registered condition, and immediately prior to penetrating an item to be sampled,
Figure 10 is a sectional view of the sampling cartridge where the sample collector has moved from its storage condition and to its sample storage container registered condition,
Figure 11 illustrates the sampling cartridge engaged with the sampling device and parts of the internal mechanism of the sampling device wherein the sampling cartridge is in its pre-use condition,
Figure 12 shows the mechanism of the sampling device with its driver having driven the sample collector towards and into the storage container.

### DETAILED DESCRIPTION OF THE INVENTION

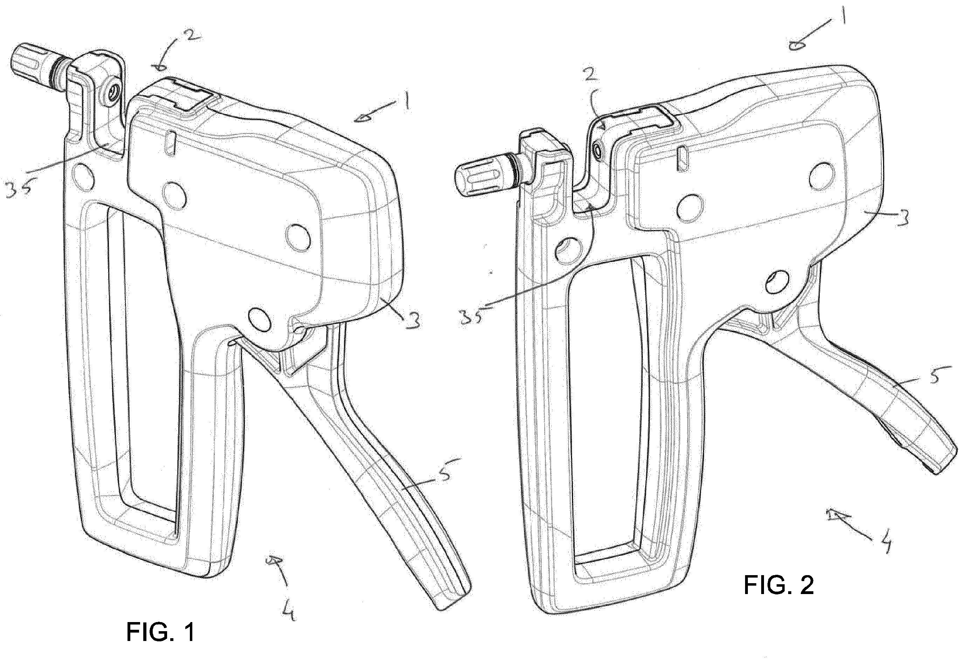

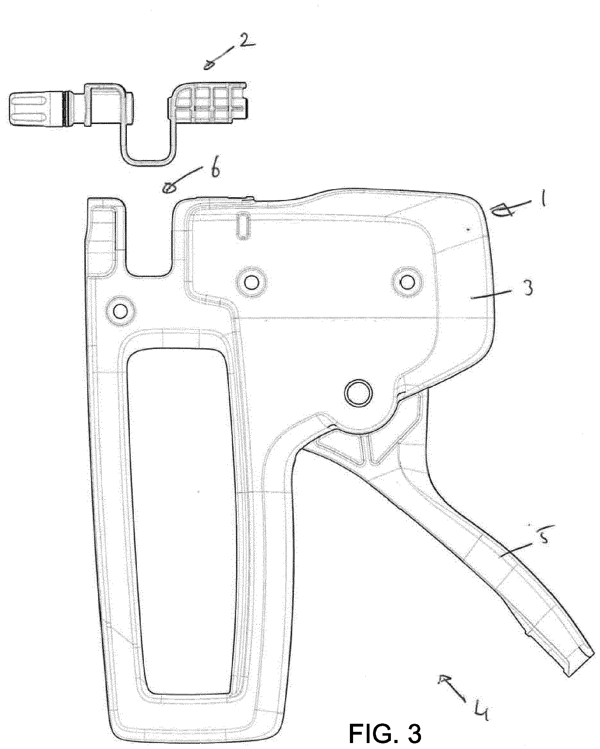

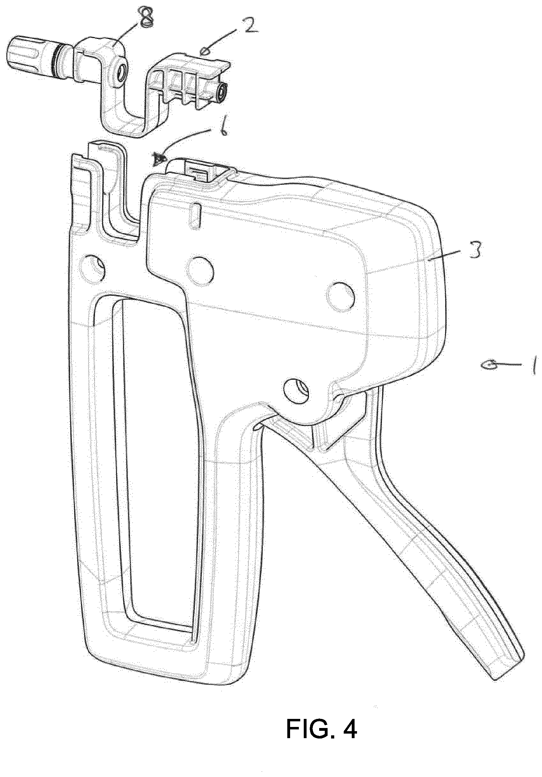

With reference to Figure 1, there is shown a sampling device (1) and a sampling cartridge (2). The sampling device includes a body (3) that includes a handle region (4) including a trigger (5). The trigger can be manipulated by a user to cause a sample to be taken and which will hereinafter be described in more detail. The sampling device (1) includes a sampling cartridge receptacle region (6) as can be seen in Figures 3 and 4.

The receptacle region is able to receive the sampling cartridge in a removable manner. Once received at the receptacle region, the cartridge is securely held by the sampling device. The sampling device can receive multiple cartridges in succession. The receptacle is adapted and configured together with the sampling cartridge (2), to allow the sampling cartridge to register with the body (3) of the sampling device (1) in a manner to present the sampling components of the sampling cartridge in an appropriate location relative to the driver (7) of the sampling device (1). In the preferred form, the receptacle region (6) and the sampling cartridge (2) are shaped and configured so that a snap-fit or close tolerance or interference fit relationship can be established between the body (3) and the sampling cartridge (2) when the sampling cartridge is full registered at the receptacle region (6) of the body (3). Such a relationship will ensure that the sampling cartridge is sufficiently securely engaged to the body (3) yet removeable therefrom upon a pulling or prying force being applied such as by a user's hand or fingers. This may be important because once loaded with the sampling device (1), the cartridge should remain engaged therewith until a sample has been taken whereafter the sampling cartridge can then be removed by the user from the body (3). The sampling cartridge loaded sampling device (1) may be dropped or placed on a surface or hung from a peg or otherwise maneuvered, prior to a sampling being taken, and it is therefore important for the sampling cartridge to remain securely engaged with the sampling device when so loaded to the sampling device. It is envisaged that a tool may also be used to remove the cartridge.

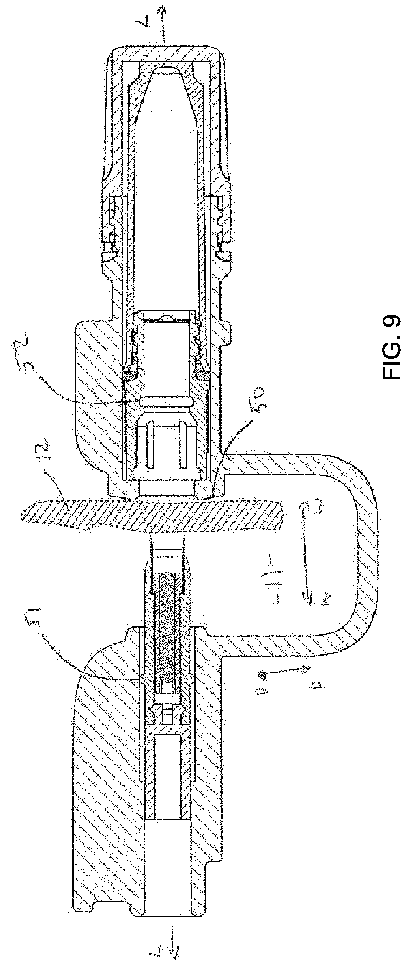

The sampling cartridge (2) preferably comprises of a sampling cartridge body (8) that in a pre-use condition holds a sample collector (9) and a storage container (10) in a space apart condition to define a gap (11) there between. At the gap (11), as can be seen in Figure 9, an item to be sampled (12) can be placed. The item to be sampled (12) may, for example, be part of an ear of an animal. The gap (11) is sufficiently wide in the direction WW to accommodate such an item as part of an ear of an animal. It is preferably also sufficiently deep in direction DD so that, for example, the ear of an animal can be located in the gap (11) and be aligned to the direction LL of the sampling components that will hereinafter be described, of the sampling cartridge (2). The gap is preferably U-shaped.

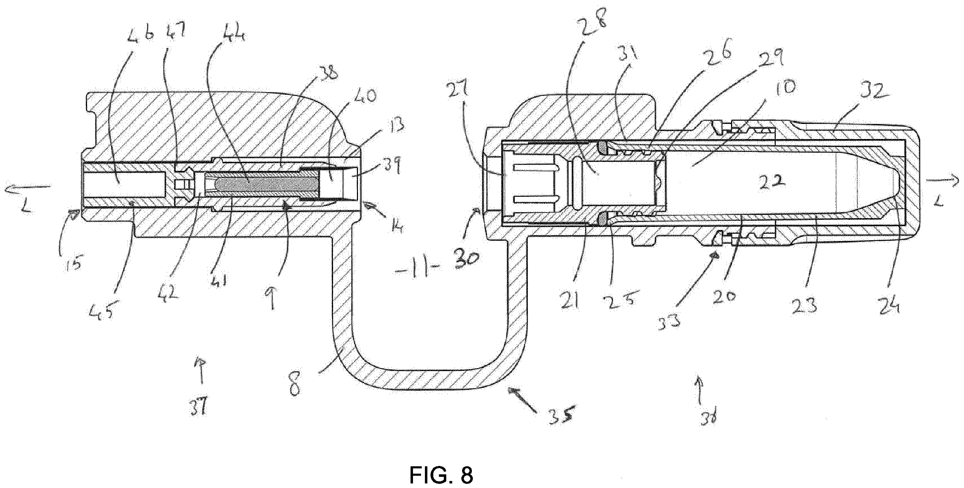

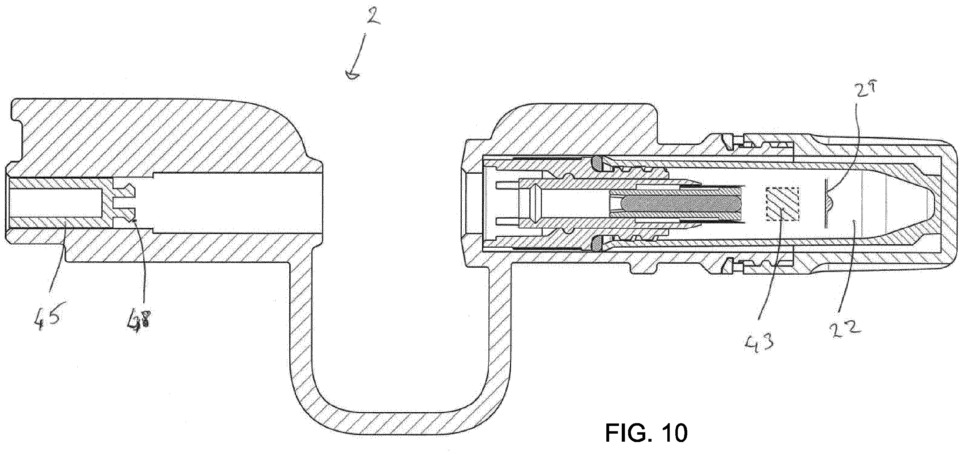

The sampling components of the sampling cartridge primarily include the sample collector (9) and the storage container (10). The sampling container body (8) preferably holds these two components in alignment with each other in the pre sampling condition. In the preferred form, the sample collector is of an elongated form and as such the sampling collector body preferably includes a bore in which the sample collector is located when in the pre sampling condition. Preferably the sample collector (9) is completely located within the bore (13) of the sample collector body (8). This can be seen in Figure 8. It is less desirable for part of the sample collector to be protruding from the bore because of the prospect then of pre sampling contamination of the sample collector (9). The bore at a gap end (14) may be sealed by way of a plug or a foil or the like to enclose the sample collector within the bore. At the opposed end (15) to the bore (13), a seal may also be provided. In the preferred form, a snug-fit relationship exists between the body of the sampling cartridge at the bore (13) and the sample collector (9) so that the bore can guide the movement of the sampling cartridge as it moves from its pre-use condition as shown in Figure 8 to its sample storage container registered condition as shown in Figure 10. Preferably the shape and configuration of the sampling cartridge body and the storage container and sample collector is such that the sample collector (9) is guided by the bore (13) and/or by the storage container during its movement from its pre-use stored condition to its sample storage container registered condition.

At the other side of the gap (11) to where the sample collector (9) is located in the pre sampling and stored condition, is a storage container (10). The storage container (10) is removably engaged to the sampling cartridge body (8). The storage container (10) preferably includes a storage tube (20) and a removable cap (21) that is removably engaged to the storage tube (20). In the preferred form the removable cap (21) is threadingly engaged to the storage tube (20). However, in alternative forms the storage container (10) may be provided as a unitary body where no separate storage tube and cap are provided. The storage tube (20) preferably includes a containment region (22) at where ultimately a sample that has been collected by the sample collector is placed during the sampling process. The containment region (22) may include an additive such as a desiccant or the like (not shown). The containment region (22) is bounded by a tube side wall (23) and the end wall (24) at the mouth opening (25) of the storage tube (20) is where the cap (21) is located. As can be seen such location is by way of a threaded engagement (26). The storage container, preferably defined by the cap (21) when provided, includes an opening (27) via which the sample collector (9) can enter the storage container (10).

The opening (27) is sufficiently large to receive the sample collector (9). The opening (27) leads to a passage (28) of the cap (21). The passage (28) leads to the containment region (22). The passage may be occluded, in the pre sampling condition by a frangible web (29) that extends across the passage (28). The frangible web (29) is preferably of a construction that will allow for the sample collector (9) to sever it at least partially from the cap (21) and thereby open the containment region (22) and allow for at least part of the sample collector to enter into the containment region (22) or at least allow for the sample collected by the sample collector to be dispensed into the containment region (22). The frangible web (29), in the pre sampling condition helps ensure that no contaminants can enter into the containment region (22). In addition, or alternatively, a plug, or seal or foil may be placed at the opening (27) of the storage container. Alternatively, and in the configuration as shown where the storage container (10) is held by the sample collected body (8), the sample collected body may include an opening (30) that leads to the storage container (10). The opening (30) may be sealed in the pre sampling condition of the sampling cartridge (2) such as by way of a plug or foil seal, to prevent contaminants from reaching the opening (27) of the storage container (10). As can be seen in Figure 8 for example, the storage container (10) is preferably also of an elongate form in direction LL. In the preferred form, the sampling cartridge body (8) also includes a bore (31) in which at least part of the storage container (10) can be located and preferably snugly supported thereby. The bore (31) is preferably of a size so that it can rigidly and snugly hold, yet allow for removal, of the storage container (10) from the sampling cartridge body (8).

In the preferred form, the bore is capped by a cap (32) that can secure on to the sampling cartridge body (8). The cap (32) preferably encloses the storage container (10) within the sampling cartridge body (8). The cap (32) is removable from the sampling cartridge body (8) and preferably is threadingly engaged therewith. A tamper evident ring (33) is preferably provided so that an indication of the removal of the cap (32) from the sampling cartridge body can be indicated. The tamper evident ring (33) may be part of the cap (32) or part of the sampling container body (8). Alternatively, it may be a foil seal that is located over the interface between the cap (32) and the sampling container body (8).

Once the cap (32) has been removed from the sampling cartridge body, the storage container can be drawn out from the bore (31) to then remove the storage container from the sampling cartridge body. This will typically happen when sampling has occurred, the sample carrying sample collector (9) having been driven into the storage container (10) and retained thereby with the sample in the containment region (22). Such removal may occur, for example, at a laboratory. This means, for example, once a sample has been collected from an animal (e.g. from the ear of an animal) by a farmer, the entire sampling cartridge still retaining the storage container and sample holding sample collector may then be shipped to a laboratory as a single unit. At the laboratory, the cap (32) may be removed to gain access to the sample holding storage container and this may then be opened to gain access to the sample for analysis.

The sampling cartridge body (8) includes a trough region (35) that bridges the gap (11). The trough region (35) connects the storage container holding region (36) and the sample collector holding region (37) together. A bridge section extends across the trough region to connect the storage container holding region (36) and the sample collector holding region (37) together. The trough region (35) also provides a secondary purpose. During the process of sampling, hair and/or blood from the animal may shed from the animal and/or the sample collected from the animal. The trough region (35) provides shielding to the body (3) of the sampling device from such contaminants. This will help keep the body (3) of the sampling device clean or cleaner than it would otherwise be.

Preferred forms of the sample collector (9) and the storage container (10) will now be described.

In a preferred form, the sample collector (9) comprises of a punch (38) that at one end presents a cutter (39). The cutter may, for example, be of a cylindrical configuration and have a leading edge that is sharp for the purposes of penetrating into the tissue of an animal. The cutter (39) may include a bore (40) at where a sample can be retained upon the penetration of the cutter into the item from which the sample is to be removed. The bore (40) can retain the so removed sample with the punch (38). The punch (38) is preferably of an elongate configuration and is preferably of a cylindrical perimeter. In the preferred form, the punch may carry a plunger (41). The plunger (41) is preferably held in a bore (42) of the punch (38). The plunger can be caused to move within this bore.

In the preferred form, in the pre sampling condition the plunger is in a retracted condition as seen in Figure 8. In this retracted condition, the plunger and the cutter define the bore (40). The bore (40) is hence a blind bore. In an extended condition as seen in Figure 10, the plunger has moved relative to the punch and into the bore (40). This has caused the sample (43) to be ejected from the bore (40) and into the containment region (22). In the sample storage container registered condition as seen in Figure 10, the punch has also severed the frangible web (29) to open the containment region (22) to the sample (43). In the preferred form, the plunger (41) carries an ID such as an EID (44) that may, for example, be an RFID. This RFID carries a unique identifier that is machine readable and remains associated with the plunger (41), and hence the punch (38) of the sample collector (9).

In the preferred form, the plunger (41) and the punch (38) cannot be separated.

As can be seen in Figure 38, a cross-contamination sleeve/rod (45) may be provided in a removable association with the sample collector (9). The cross-contamination sleeve/rod provides an intermediary between the driver (7) of the sampling device (1) and the sample collector (9). The driver (7) may penetrate into the bore (46) of the cross-contamination sleeve/rod (45) in a manner to isolate it from contact with the item to be sampled. This hence provides a further measure against cross-contamination that might otherwise arise from contaminants touching the driver (7) of the sampling device (1). In the preferred form, the cross-contamination sleeve/rod (45) is engaged with the punch (38) at the driving end (47) of the punch so that it can drive the sample collector from its pre-use stored condition as seen in Figure 8 to its sample storage container registered condition as seen in Figure 10. The cross-contamination sleeve/rod may be removably engaged by way of a snap fit or interference fit configuration (48) that can registered at the driving end (47) of the punch (38). It can be released upon the retraction of the driver (7) and be carried back with the driver after the sample has been taken and the sample collector is in the storage container, as can for example be seen in Figure 10. Alternatively, the cross-contamination sleeve/rod (45) may be ejected from the sampling cartridge (2) after sampling.

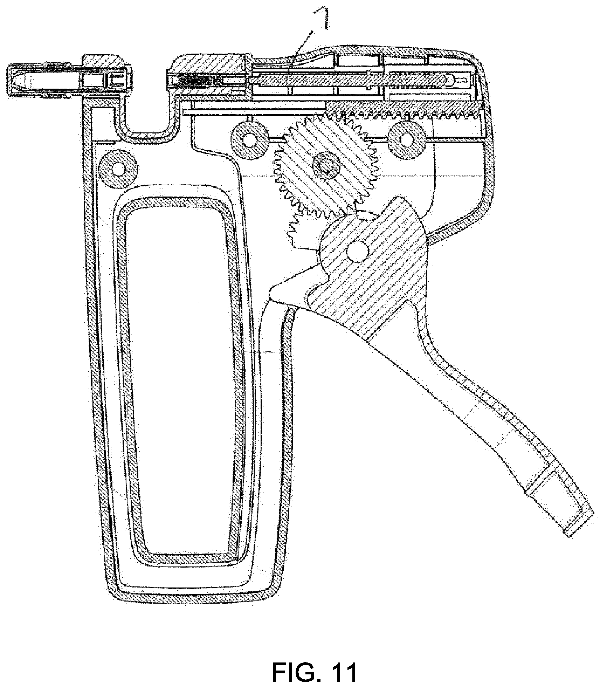

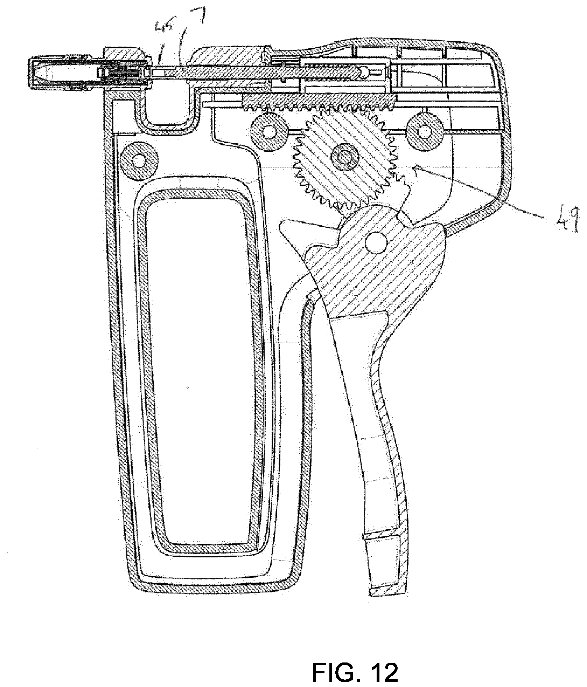

The driver (7) is preferably held relative to the body (3) in a manner to be actuable by the trigger (5) to cause it to move in a reciprocating manner between a retracted condition as seen in Figure 11, and an extended condition as seen in Figure 12. A rack and pinion-like arrangement (49) may be provided to facilitate the movement of the driver. A quick-release feature may be provided so that once the driver has been moved to its fully extended position a spring or other device may quickly retract the driver from its extended position back its retracted condition.

When a sample is being taken, the sample collector (9) may drive and/or press the item to be sampled (12) towards the storage container holding region of the sampling cartridge (2). As can be seen in Figure 9, the item to be sampled (12) is pressed against a die surface (50) of the storage container holding region (36) of the sampling cartridge body (8). The die surface (50) acts as a stop for the item to be sampled (12) as the sample collector is driven towards its sample storage container registered condition. The die surface (50) together with the cutter (39) act as a punch and die set to facilitate the removal of a sample from the item to be sampled (12). Hence in the preferred form, the opening (30) is sufficiently sized to allow for it to receive the cutter, and preferably the entire sample collector (9), yet act in a sheer fashion with the cutter (39) for the purposes of removing the sample. Hence the sizing of the opening (30) is sufficiently snug about the cutter to facilitate this sheering-like removal of the sample from the item to be sampled.

Upon a driving of the sample collector into the cap (21) of the storage container (10), the punch preferably locates very snugly within the passage (28) to thereby provide a seal to the passage (28). An interference fit ring (51) and groove (52) configuration may be provided by the punch (38) and cap (21) respectively, to facilitate such sealing. The ring and groove configuration will also help with retention of the sample collector with the storage container after the sample collector has been moved to its sample storage container registered condition, as can be seen in Figure 10.

To facilitate such snug/interference fit between the sample collector and the storage container, the material selection of one or both of the sample collector and the storage container (preferably the punch and the cap) needs to be such as to allow for such a snap-fit type relationship to be established. Therefore, preferably one or both of the punch and cap are of a relatively soft and preferably plastic material, to allow for a certain degree of deformation of features of one or both of these items to occur. Hence in the preferred form, the cap (21) is preferably of a plastics material and shape and configuration that can to some extent deform on its own, or preferably together with features of the sample collector to establish a snug, and preferably sealed relationship between the cap and the sample collector at the passage (28). In the preferred form, to ensure an efficient punch and die set-like relationship is established between the cutter (39) and the die surface (50), the die surface (50) is defined by a material which is rigid and preferably will not deform. In the preferred form, the die surface is part of the storage container body. Preferably, the die surface is of a material that is harder than that of the cap (21) of the storage container (10). Hence the present invention allows for an efficient punch and die set-like features to be provided for the purposes of taking the sample, without such being compromised by needing to use a material that may be softer of the cap of the storage container (10). Hence in the preferred form, the storage container cap is of a softer plastics material than the material used at the die surface (50) (preferably the material used for the storage container body). It will be appreciated that a separate insert may be provided to the storage container body to act as the die surface. A co-molding of different plastics materials may be possible, or an insert of, for example, a metal insert may be provided for the purposes of providing the die surface (50).

One or both of the sampling cartridge body (8) and tube (20) may include a unique identifier. For example, a barcode or number or alphanumeric string may be provided on a surface of the sampling cartridge body. Alternatively, or additionally, an EID component may be provided on the sampling cartridge body. In addition, a unique barcode or other visual identifier or an EID may also be provided on the tube (20). This may for example be provided at the end wall (24) of the storage tube (20). Any one or more, and preferably all of the identifiers mentioned herein (EID/barcode or other) are preferably matched such a pre-matched to each other. It may be of the same identification or different identification that are matched with each other. The sample collector ma also include such identifier. Preferably the identifiers relate to each other. They may be identical identifiers or via a database or other related to each other.

Upon the taking of a sample from an animal, a reading of an identifier of the animal (such as an RFID or visual ID of an ear tag of the animal) may be matched to the identifiers of the sampling cartridge (2) and/or tube and/or sample collector. This allows for a matching between the animal identification and the sample to be established.

In a laboratory, such matching can then verify whether the sample in the storage container is a valid sample that matches that of the animal from which the sample has been taken. The reading of the animal's identification may occur before, during or after the sample being taken from the animal.

In the preferred form, the cap (32) is of a transparent material. This allows for a visual inspection to occur to ensure that a sample is within the storage tube. Hence preferably the storage tube is also at least partially transparent.

### CLAUSES

1. A sampling cartridge comprising a sampling cartridge body that includes a storage container holding region at where a storage container is or is able to be located and a sample collector holding region at where a sample collector is or is able to be located, wherein the body in use holds the sample collector and storage container in alignment with each other in a pre-sampling condition spaced apart with a sufficient gap to allow part of an item to be sampled to locate at said gap.
2. A sampling cartridge as in clause 1 adapted and configured to be loadable and unloadable to and from a sampler device that includes a driver actuable to cause said in alignment sample collector to move from sample collector holding region across the gap and into the sample storage container.
3. A sampling cartridge as in clause 2 wherein the sampling cartridge body is adapted and configured to shield the sampler device at the gap from contact with said item to be sampled when placed at the gap.
4. A sampling cartridge as in clause 2 wherein the sampling cartridge body is adapted and configured to shield the sampler device at the gap from contact with said item to be sampled when placed at the gap, and any exudates expressed or expelled from said item during sampling.
5. A sampling cartridge as in any one of clauses 1 to 4 comprising a bridge section extending between said storage container holding region and said sample collector holding region, said bridge section spanning said gap.
6. A sampling cartridge as in clause 5 when dependent on clause 3 or 4 wherein said bridge section provides said shield function.
7. A sampling cartridge as in clause 5 or 6 wherein said bridge section is the only connection of the cartridge between said storage container holding region and said sample collector holding region.
8. A sampling cartridge as in any one of clauses 5 to 7 wherein said bridge section defines a trough to said gap.
9. A sampling cartridge as in any one of clauses 5 to 8 wherein the gap is substantially U shaped, including two facing side walls and a base defined by said bridge section and said storage container holding region and said sample collector holding region.
10. A sampling cartridge as in any one of clauses 1 to 9 wherein the gap is substantially U shaped.
11. A sampling cartridge as in any one of clauses 1 to 10 wherein said storage container holding region and said sample collector holding region are contiguous respective opposed sides of said gap.
12. A sampling cartridge as in any one of clauses 1 to 11 wherein said storage container holding region comprises a cavity of said sampling cartridge body in which at least part of said storage container is located.
13. A sampling cartridge as in clause 12 wherein said cavity is a bore.
14. A sampling cartridge as in clause 12 or 13 wherein a passage leads from said cavity to said gap and via which sample collector can move to engage with said storage container.
15. A sampling cartridge as in clause 14 wherein the passage is blocked pre-sampling.
16. A sampling cartridge as in clause 15 wherein the passage is blocked by a removable seal.
17. A sampling cartridge as in clause 16 wherein the removable seal is a film, preferably able to be ruptured and/or peeled away from the passage to expose the passage to said gap.
18. A sampling cartridge as in any one of clauses 14 to 17 wherein the passage includes a mouth opening presented at said gap at where said sample collector enters the passage, upon the taking of a sample from an item to be sampled placed at the gap, to advance for engagement via on opening of and into said storage container.
19. A sampling cartridge as in clause 18 wherein the material about said mouth is harder than said material of the storage container about its said opening.
20. A sampling cartridge as in clause 18 or 19 wherein at said mouth opening said sampling cartridge body together with said sample collector act as a punch and die set, the mouth opening acting as the die acting in shear with the sample collector acting as said punch to facility the removal of a sample from the item to be sampled.
21. A sampling cartridge as in clause 20 wherein said mouth opening is of a shape and configuration to allow said sample collector to enter into said passage in a snug fit manner.
22. A sampling cartridge as in any one of clauses 14 to 21 wherein passage extends between its said opening and said cavity at or adjacent where an opening of said storage container is presented.
23. A sampling cartridge as in any one of clauses 12 to 22 wherein said cavity is closed by a removable closure that together with said cavity encloses said storage container in said sampling cartridge body.
24. A sampling cartridge as in clause 23 wherein said removable closure is able to be removed to open said cavity and expose said storage container to allow said storage container to be removed from said sampling cartridge body.
25. A sampling cartridge as in clause 23 or 24 wherein said removable closure is engaged to said sampling cartridge body in a tamper evident manner.
26. A sampling cartridge as in any one of clauses 23 to 25 wherein said removable closure is threadingly engaged to said sampling cartridge body.
27. A sampling cartridge as in any one of clauses 1 to 26 wherein said sampling cartridge body includes a second cavity in which said sample collector is at least partially located, pre-sampling.
28. A sampling cartridge as in clause 27 wherein said sample collector is entirely located in said second cavity.
29. A sampling cartridge as in clause 27 or 28 wherein said second cavity includes an opening to said gap via which said sample collector can pass from said cavity towards said storage container during sampling.
30. A sampling cartridge as in clause 29 wherein said opening of said second cavity is sealed by a removable seal (e.g. a film seal of cap or plug).
31. A sampling cartridge as in clause 29 or 30 wherein said second cavity is fully sealed pre sampling to enclose said sample collector therein.
32. A sampling cartridge as in any one of the preceding clauses wherein the body carries only one sample collector and one sample storage container.
33. A sampling cartridge as in any one of the preceding clauses wherein at least one of the components selected from the following list:
   a. Removable closure,
   b. Storage container (preferably the storage tube),
   c. Sample collector (preferably the plunger)
   d. Sampling cartridge body,
      comprises at least one form of visible and/or machine readable identification.
34. A sampling cartridge as in clause 33 wherein the identification is a unique identification.
35. A sampling cartridge as in clause 33 or 34 wherein the identification is not the same on each of said components but are preferably matched.
36. A sampling cartridge as in any one of clauses 33 to 35 wherein the identification is provided in the form selected from one of more of an electronic identification (EI D) or visible identification such as a bar code, number, QR code, alphanumeric string or the like.
37. A sampling cartridge as in any one of the preceding clauses wherein the sample collector comprises a punch presenting at one end a cutter to cut and hold a sample from the item as it passes there through, the punch having a bore at where a sample, once cut is able to locate with said punch.
38. A sampling cartridge as in clause 37 wherein said sample collector further comprises a plunger slideably supported at said bore to be able to move between a retracted position and an advanced position relative said punch, the movement from the retracted position to the advanced position causing, in use, the cutter held sample to be displaced from the cutter.
39. A sampling cartridge as in clause 37 or 38 wherein the sample collector is generally of circular cross section and elongate and straight, the cutter located at one end of the collector.
40. A sampling cartridge as in any one of clauses 37 to 39 wherein the punch is generally of circular cross section and elongate and straight, the cutter located at one end of the collector.
41. A sampling cartridge as in any one of clauses 37 to 40 wherein the plunger protrudes from the end of the punch opposite the cutter.
42. A sampling cartridge as in any one of clauses 37 to 41 the plunger is rod shaped.
43. A sampling cartridge as in any one of clauses 37 to 42 wherein the punch is to seal the opening (or passage extending therefrom) of a sample storage container with which the sample collector is to engage after the sample has been taken.
44. A sampling cartridge as in any one of clauses 1 to 43 wherein the storage container includes a mouth opening leading to a storage region of the storage container, the mouth opening able to receive the sample collector after the sample collector has been driven through the item and is carrying a sample.
45. A sampling cartridge as in clause 44 wherein the storage container is adapted and configured to retain the sample collector (preferably the punch) once received at said mouth opening in a manner to snugly hold the sample collector to said storage container (preferably sealing the mouth opening).
46. A sampling cartridge as in any one of clauses 44 to 45 wherein said storage container (preferably at or adjacent said mouth opening) includes a surface or surfaces that allow the sample collector to seal the containment region of the storage container.
47. A sampling cartridge as in any one of clauses 1 to 46 wherein said storage container comprises a container body defining a containment region and a cap removably engaged to the container body, a mouth opening to said container body provided at a passage defined by said cap.
48. A sampling cartridge as in clause 47 wherein said passage and said sample collectors are adapted and configured to engage in a snap-fit manner to retain the sample collectors with the cap and seal the containment region.
49. A method of taking and storing a sample from an item using the sampling cartridge as in any one of clauses 1 to 48 comprising:
   a. loading the sampling cartridge to a sampler device so that the sample collector and storage container are in alignment with a driver of said sampler device, the driver to act, directly or indirectly on said sample collector,
   b. placing a part of an item to be sampled in said gap so that part of the item is also in alignment,
   c. causing the driver to move in a driving direction to drive said sample collectors from its holding region, across the gap and towards said storage container so that said sample collector is driven into the storage container so that said punch becomes held by said storage container,
   d. causing the driver to move in a direction opposite said driving direction.
50. The method as in clause 49 wherein said identification carried by or of the item from which the sample is taken is recorded at the time of taking of the sample.
51. The method as in clause 50 wherein the identification is matched to identification of said sampling cartridge.
52. The method as in any one of clauses 49 to 51 wherein said sampling cartridge is removed from the sampling device after the sample has been removed from the item to be taken, the sampling cartridge carrying the sample collector engaged with the storage container at one side of the gap.
53. The method as in clause 52 wherein the removed sampling cartridge is then sent to a laboratory for sample analysis.
54. The method as in clause 53 wherein the identification and the item and the identification of the sampling cartridge are reviewed after sampling to determine these correspond to the matched identification.
55. A kit of a sampler and sampling cartridge as in any one of clauses 1 to 48 wherein the sampler comprises an actuable driver and a receptacle configured to releaseably receive said cartridge to said sampler said driver presented and actuable to cause said in alignment sample collector to move from sample collector holding region across the gap and into the sample storage container.
56. A kit as in clause 55 wherein the sampler comprises a U shape receptacle at where the cartridge is receivable.
57. A kit as in clause 55 or 56 wherein the sampling cartridge body is adapted and configured to shield the sampler at the gap from contact with said item to be sampled when placed at the gap, and any exudates expressed or expelled from said item during sampling.
58. A sampler for use with the cartridge as in any one of clauses 1 to 48.

## Claims

1. A sampling cartridge comprising a sampling cartridge body that includes a storage container holding region at where a storage container is or is able to be located and a sample collector holding region at where a sample collector is or is able to be located, wherein the body in use holds the sample collector and storage container in alignment with each other in a pre-sampling condition spaced apart with a sufficient gap to allow part of an item to be sampled to locate at said gap.

2. A sampling cartridge as claimed in claim 1 adapted and configured to be loadable and unloadable to and from a sampler device that includes a driver actuable to cause said in alignment sample collector to move from sample collector holding region across the gap and into the sample storage container.

3. A sampling cartridge as claimed in claim 2 wherein a) the sampling cartridge body is adapted and configured to shield the sampler device at the gap from contact with said item to be sampled when placed at the gap, or b) the sampling cartridge body is adapted and configured to shield the sampler device at the gap from contact with said item to be sampled when placed at the gap, and any exudates expressed or expelled from said item during sampling.

4. A sampling cartridge as claimed in any one of claims 1 to 3 comprising a bridge section extending between said storage container holding region and said sample collector holding region, said bridge section spanning said gap, wherein said bridge section provides said shield function.

5. A sampling cartridge as claimed in any one of claims 1 to 4 wherein the gap is substantially U shaped.

6. A sampling cartridge as claimed in any one of claims 1 to 5 wherein said storage container holding region and said sample collector holding region are contiguous respective opposed sides of said gap.

7. A sampling cartridge as claimed in claim any one of claims 1 to 6 wherein said storage container holding region comprises a cavity of said sampling cartridge body in which at least part of said storage container is located.

8. A sampling cartridge as claimed in claim 7 wherein a passage leads from said cavity to said gap and via which sample collector can move to engage with said storage container.

9. A sampling cartridge as claimed claim 8 wherein the passage includes a mouth opening presented at said gap at where said sample collector enters the passage, upon the taking of a sample from an item to be sampled placed at the gap, to advance for engagement via on opening of and into said storage container.

10. A sampling cartridge as claimed in claim 9 wherein the material about said mouth is harder than said material of the storage container about its said opening.

11. A sampling cartridge as claimed in claim 9 or 10 wherein at said mouth opening said sampling cartridge body together with said sample collector act as a punch and die set, the mouth opening acting as the die acting in shear with the sample collector acting as said punch to facility the removal of a sample from the item to be sampled.

12. A sampling cartridge as claimed in any one of the preceding claims wherein at least one of the components selected from the following list:
a. Removable closure,
b. Storage container,
c. Sample collector,
d. Sampling cartridge body,
comprises at least one form of visible and/or machine readable identification.

13. A method of taking and storing a sample from an item using the sampling cartridge as claimed in any one of claims 1 to 12 comprising:
a. loading the sampling cartridge to a sampler device so that the sample collector and storage container are in alignment with a driver of said sampler device, the driver to act, directly or indirectly on said sample collector,
b. placing a part of an item to be sampled in said gap so that part of the item is also in alignment,
c. causing the driver to move in a driving direction to drive said sample collectors from its holding region, across the gap and towards said storage container so that said sample collector is driven into the storage container so that said punch becomes held by said storage container,
d. causing the driver to move in a direction opposite said driving direction.

14. The method as claimed in claim 13 wherein a) said identification carried by or of the item from which the sample is taken is recorded at the time of taking of the sample, or b) the identification is matched to identification of said sampling cartridge.

15. A kit of a sampler and sampling cartridge as claimed in any one of claims 1 to 12 wherein the sampler comprises an actuable driver and a receptacle configured to releaseably receive said cartridge to said sampler said driver presented and actuable to cause said in alignment sample collector to move from sample collector holding region across the gap and into the sample storage container.
